# EUROPEAN PATENT SPECIFICATION

(11) **EP 1 211 932 B1**
(45) Date of publication and mention of the grant of the patent: **11.03.2009**
(21) Application number: 00958098.6
(22) Date of filing: 08.09.2000
(51) Int. Cl.: A01K 67/027

(54) **A METHOD OF TRANSFERRING SPERMATOGONIAL GERMLINE CELLS IN COCKS**
VERFAHREN ZUM TRANSFERIEREN VON SPERMATONIALEN KEIMZELLEN BEI HÄHNEN
PROCEDE DE TRANSFERT DE CELLULES GERMINALES SPERMATOGONIALES CHEZ LE COQ

(30) Priority: 08.09.1999 CZ 318699
(43) Date of publication of application: 12.06.2002
(73) Proprietor: Biopharm, Výzkumný ústav biofarmacie a Veterinárních léciv a.s., 254 49 Jílové u Prahy (CZ)
(72) Inventor: TREFIL, Pavel, 104 00 Praha 10 (CZ); KOTRBOVÁ- MICÁKOVÁ, Alena, 341 01 Horazd´ovice (CZ)
(74) Representative: HOFFMANN EITLE
(86) International application number: PCT/CZ2000/000064
(87) International publication number: WO 2001/017344

(56) References cited:
- BENOIT J ET AL: "GREFFES INTERRACIALES DE TESTICULES CHEZ LES GALLINACES. TESTOTESTES FERTILES OBTENUS PAR GREFFE DE TESTICULES DE POUSSINSWYANDOTTE BLANC DANS LES TESTICULES STERILISES PAR LES RAYONS X DE COQS RHODE ISLAND RED" COMPTES RENDUS DES SEANCES DE LA SOCIETE DE BIOLOGIE ET DE SES FILIALES, XX, XX, vol. 162, no. 4, 23 November 1968 (1968-11-23), pages 838-842, XP000998009 ISSN: 0037-9026
- BRINSTER R L ET AL: "SPERMATOGENESIS FOLLOWING MALE GERM-CELL TRANSPLANTATION" PROCEEDINGS OF THE NATIONAL ACADEMY OF SCIENCES OF USA, NATIONAL ACADEMY OF SCIENCE. WASHINGTON, US, vol. 91, November 1994 (1994-11), pages 11298-11302, XP000993002 ISSN: 0027-8424
- BRINSTER R L ET AL: "GERMLINE TRANSMISSION OF DONOR HAPLOTYPE FOLLOWING SPERMATOGONIAL TRANSPLANTATION" PROCEEDINGS OF THE NATIONAL ACADEMY OF SCIENCES OF USA, NATIONAL ACADEMY OF SCIENCE. WASHINGTON, US, vol. 91, November 1994 (1994-11), pages 11303-11307, XP000993003 ISSN: 0027-8424 cited in the application
- AIGE-GIL V; SIMKISS K : "STERILIZATION OF AVIAN EMBRYOS WITH BUSULPHAN" RESEARCH IN VETERINARY SCIENCE , vol. 50, 1991, pages 139-144, XP000998022 cited in the application
- VICK L ET AL: "GERM-LINE CHIMERAS CAN PRODUCE BOTH STRAINS OF FOWL WITH HIGH EFFICIENCY AFTER PARTIAL STERILIZATION" JOURNAL OF REPRODUCTION AND FERTILITY, JOURNALS OF REPRODUCTION AND FERTILITY LTD, GB, vol. 98, no. 2, July 1993 (1993-07), pages 637-641, XP000998004 ISSN: 0022-4251
- AIGE-GIL V ET AL: "STERILISING EMBRYOS FOR TRANSGENIC CHIMAERAS" BRITISH POULTRY SCIENCE, LONGMAN GROUP, GB, vol. 32, 1991, pages 427-438, XP000994670 ISSN: 0007-1668
- WISHART G J ET AL: "EFFECT OF GAMMA-RADIATION ON FOWL SPERM FUNCTION IN VITRO AND IN VIVO" JOURNAL OF REPRODUCTION AND FERTILITY, JOURNALS OF REPRODUCTION AND FERTILITY LTD, GB, vol. 75, no. 2, November 1985 (1985-11), pages 617-622, XP000998034 ISSN: 0022-4251
- VICK L ET AL: "TRANSGENIC BIRDS FROM TRANSFORMED PRIMORDIAL GERM CELLS" PROCEEDINGS OF THE ROYAL SOCIETY OF EDINBURGH. SECTION B, BIOLOGICAL SCIENCES, ROYAL SOCIETY OF EDINBURGH, EDINBURGH, GB, vol. 251, no. 1332, 22 March 1993 (1993-03-22), pages 179-182, XP000997980 ISSN: 0269-7270

## Description

### Field of the Invention

The present invention relates to a method of transferring spermatogonial germline cells for transfer of genetic information in fowl strain.

### Background of the Invention

Biotechnological research in the field of transgenic fowl is far less successful then the one in the field of the mammals. The main cause of this state is the unique reproductive apparatus of the birds.

In difference to the mammals, the bird's zygote is not accessible and it is connected with an extraordinarily large yolk sac. The yolk mass prevents identification of pronuclei, in the process of fertilization a polyspermia takes place and it is not possible to determine which protonucleus participated in the embryo formation. Therefore, e.g. the direct microinjection into the protonucleus used in case of mice is nearly impossible in case of fowl. Therefore, in the fowl field, activity was directed to manipulation with the not yet differentiated fowl cells of whatever kind, i.e. blastodermal, primordial gonocytes or germline, spermatogonial cells.

Use of germline, spermatogonial cells in construction of transgenic constructs of mice was disclosed at first by Brinster, L.R. and Avarbock, M.R. (1994) Germline Transmission of Donor Haplotype Following Spermatogonial Transplantation, Proc. Natl. Acad. Sci. USA, 91, 11303-11307.

Virtually, it is a new field of possible germline chimaera production consisting in partial or complete destruction of the reproductive cells in testicles of a creature and in a repeated re-colonisation of the testicles by germ cells of another creature. Application of this method on fowl was not disclosed.

The first step in mastering the above technique is the testicles sterilization of an acceptor because destruction of such germline spermatogonial cells (sperm progenitors) is not easy. The use e.g. of busulphan is not entirely corresponding, and after its use, only partial sterilization of the donor takes place (see Vick, L., Luke, G., Simkiss, K. (1993) Germline Chimaeras Can Produce Both Strains of Fowl with High Efficiency after Partial Sterilization; J. Reprod. Fertil., 98, 637 - 641. On the other side, use of a higher dose brings about a total retardation of the animal what is unwanted in any case (see Smýkalová, S., Kotrbová, A., Trefil, P. (1998) Effect of Busulphan on Growth and Development of the Chicken Embryos, Vet. Med.-Czech, 43, 105-109).

The other necessary step of a successful application of this method is mastering the transfer of the germline spermatogonial cells into the acceptor, i.e. the transfer of those spermatogonial cells that build in functionally and re-colonize testicles of an acceptor, after their transfer, and subsequently produce fertilizing sperms. At this time this brings many difficulties and ambiguous results.

### Summary of the Invention

The above mentioned drawbacks are avoided in case of a method of transgenic fowl construction, preferably by transfer of the germline, spermatogonial cells, according to the present invention which method comprises irradiating the testicles only of an acceptor cock with gamma rays repeatedly, whereafter, foreign germline spermatogonial cells of a donor cock are implanted into the undamaged sterile testicles of said acceptor cock within an interval of 60 days to 2 years after the last irradiation dose of said testicles, whereby, said testicles continue in producing fertilizing sperms that store the whole genetic information of the donor cock. After insemination of hens by the so produced sperms, transgenic fowl chickens are hatched and provided ½ genetic information by said donor cock.

The method according to the present invention is defined in the claims; Accordingly, the method is carried out so that the testicles of the acceptor cock are irradiated with gamma rays using the absorbed dose 8 Gy 5-times over a week.

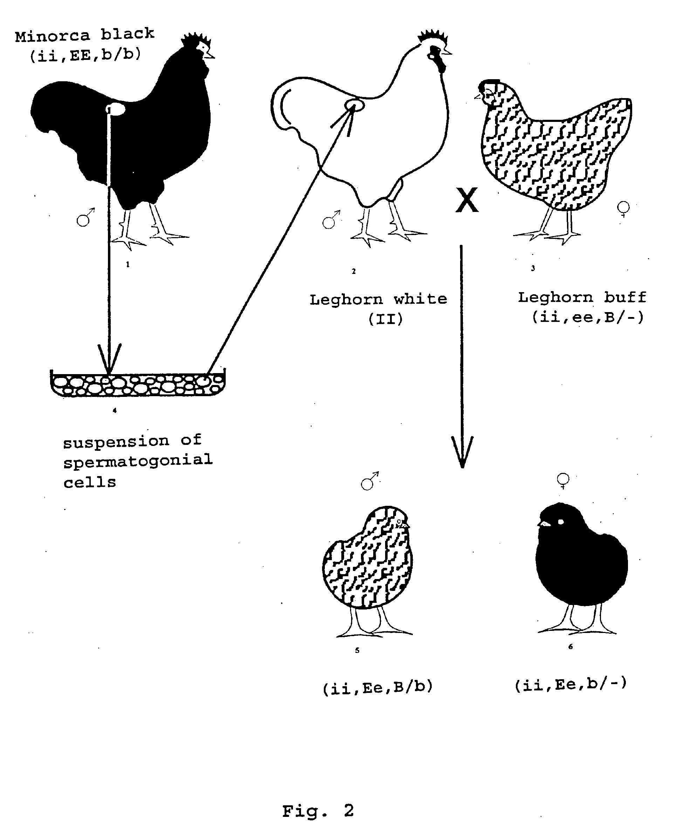

In contradiction to the other disclosed techniques, in the case of this irradiation, the original germline, spermatogonial cells are completely destroyed in the testicles of the acceptor cock, whereafter, the acceptor cock is unable to produce sperms, whereby the testicle structure including the Sertoli's cells remains preserved for the subsequent implantation of foreign germline spermatogonial cells provided by a donor cock. The so implanted germline spermatogonial cells continue in producing sperms that are able to fertilize and that store the genetic information of the germline spermatogonial cells of the donor cock (see the Diagram in Fig. 2).

Production of transgenic fowl by the method according to this invention is easy and provides results to 100 %, because the spermatogonial cells of the donor cock can be transfected by the required genetic information and so, after a successful implantation of said cells into the testicles of the acceptor cock a transgenic cock is produced, which transgenic cock will transfer to ½ the genetic information to his progeny by insemination or by natural breeding.

The fact itself that this method of transferring germline spermatogonial cells from one creature to another one is functional is extremely valuable and apart from this, it is extremely interesting especially in relation with the creation of transgenic fowl. The germline spermatogonial cells can be easily transfected in vitro by foreign genetic information before the implantation proper, which information will then be transferred to ½ to the offspring in the F1 generation. This will probably open a real way how to use fowl (fowl oviduct) as a bioreactor for production of specific proteins valuable e.g. for the pharmaceutical industry.

Therefore, a great number of tests were carried out to verify the method according to this invention, whereby, some of them are described below as examples with reference to the attached graphs and drawings.

### Brief Description of the Drawings

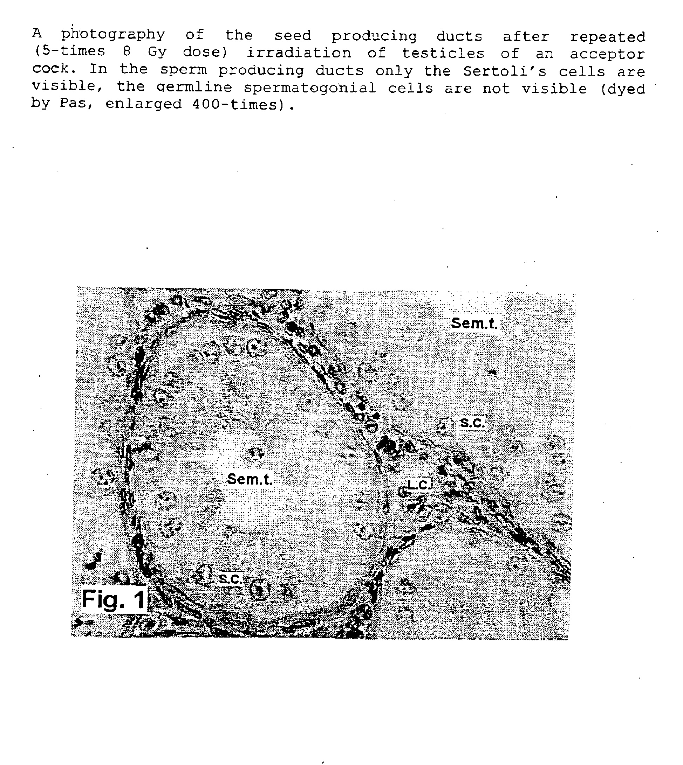

Figure 1 shows a photograph of seminiferous tubulus (Sem.t.) of repeatedly (5-times 8 Gy dose) irradiated of testes of an acceptor cock. Seminiferous epithelium are lined only by Sertoli cells(S.C.) and interstitial tissue consisting of Leydig cells(L.C.). Germline, spermatogonial cells are not visible (PAS stain, enlarged 400-times).
Figure 2 shows a diagram of the method, whereby, a Minorca black cock (ii,EE,b/b) is the donor cock 1, a Leghorn white (II) cock is the acceptor cock 2 which acceptor cock 2 is irradiated by a 8 Gy absorbed dose 5-times to stop production of own sperms. The acceptor cock 2 is then colonized by spermatogonial cells in suspension 4. The acceptor cock then inseminates a Leghorn barred hen 3 (ii,ee,B/-) to produce crossbreeds, i.e. a (ii,Ee,B/b) barred male chicken 5 and a (ii,Ee,b/-) black female chicken 6.
Figure 3 shows a graph of relation between sperms concentration (x1000/ml)(y-axis) and days elapsed after an irradiation (x-axis) 5-times by a 8 Gy absorbed dose.
Figure 4 shows a graph of relation between motility (y-axis) and days (x-axis) elapsed after irradiation 5-times by a 8 Gy absorbed dose.
Figure 5 shows a graph of relation between sperms concentration (x1000/ml)(y-axis) and days elapsed after irradiation (x-axis) by a 18 Gy absorbed dose.
Figure 6 shows a graph of relation between motility (y-axis) and days (x-axis) elapsed after irradiation by a 18 Gy absorbed dose.
Figure 7 shows a graph of relation between sperms concentration (x1000/ml)(y-axis) and days elapsed after irradiation (x-axis) by a 22 Gy absorbed dose.
Figure 8 shows a graph of relation between motility (y-axis) and days elapsed after irradiation (x-axis) by a 22 Gy absorbed dose.
Figure 9 shows a graph of relation between sperms concentration (x1000/ml) (y-axis) and days elapsed after irradiation (x-axis) by a 26 Gy absorbed dose.
Figure 10 shows a graph of relation between motility (y-axis) and days elapsed after irradiation (x-axis) by 26 Gy absorbed dose.
Figure 11 shows a graph of relation between sperms concentration (x1000/ml) (y-axis) and days elapsed after irradiation (x-axis).
Figure 12 shows a graph of relation between motility (y-axis) and days elapsed after irradiation (x-axis).

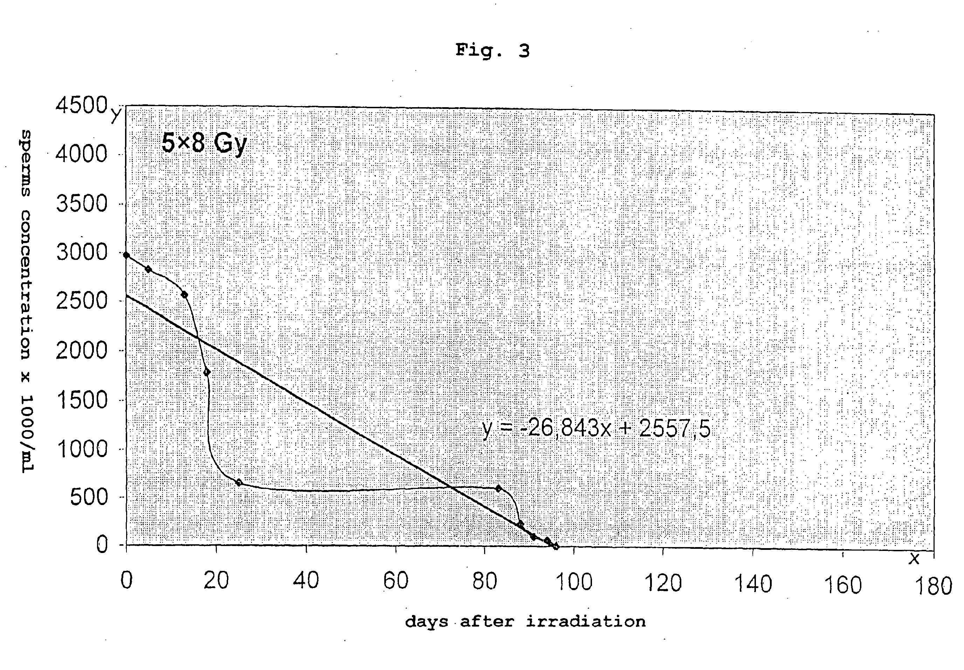

For the comparison of spermatozoa motility and spermatozoa concentration (from Fig.3 to Fig.12) between treated groups, the linear regression model was used (Baloui(1966), Like and Machek(1983)). From the slope of on the gradient line it is possible to characterize the changes of the values in the parameters. '

Spermatozoa motility of was subjectively estimated according to Trefil, 1995.Selection of chicken sires for semen production in cages. Book of abstracts of the 46^{th} Annual Meeting of the European Association for Animal Production, Prague, September,165. Very good motility has a no. 5 rating and very poor motility no.1.

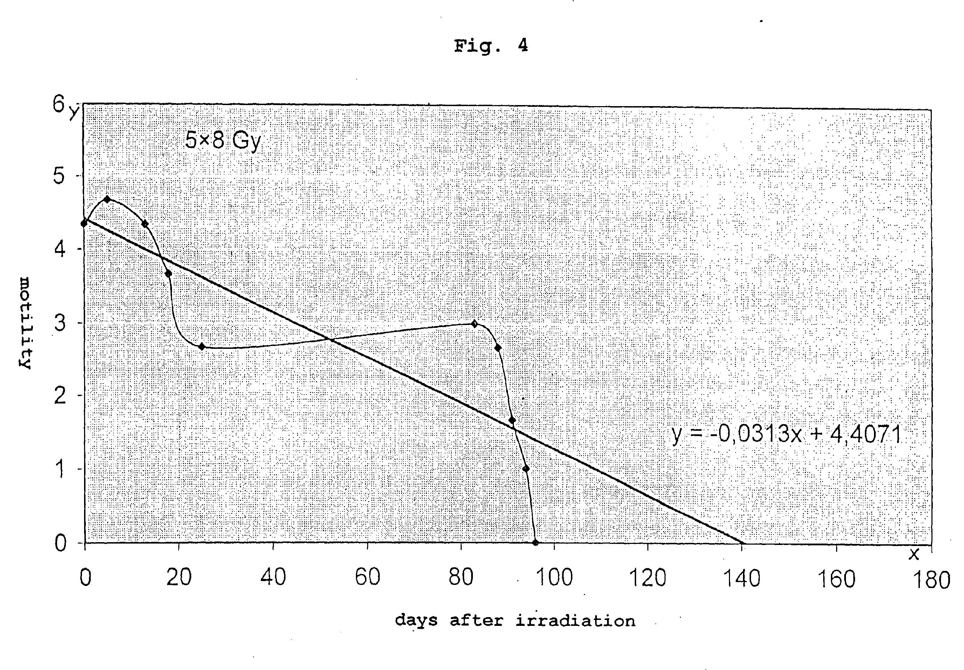

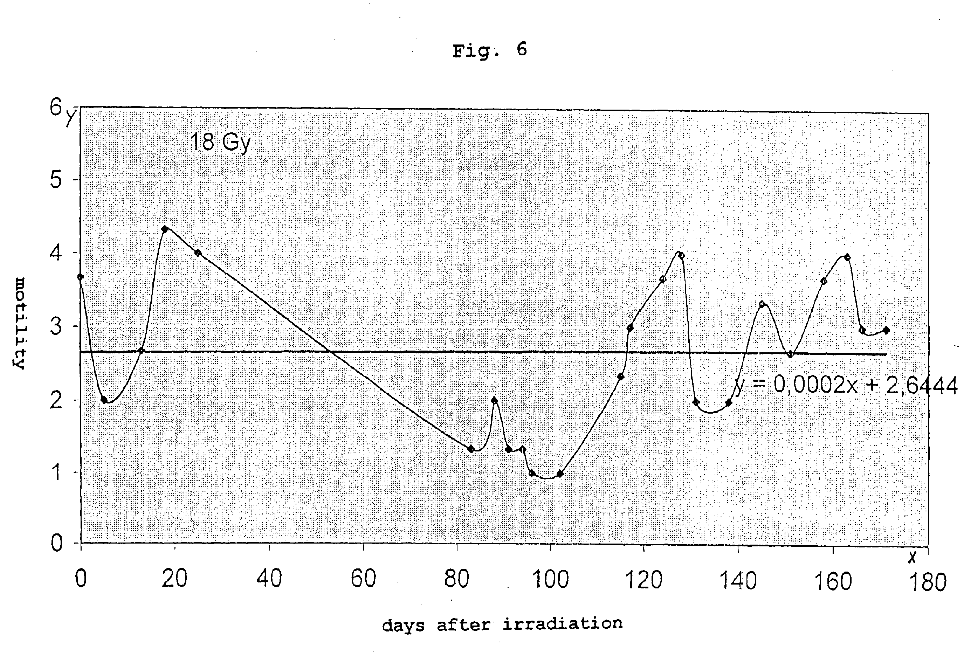

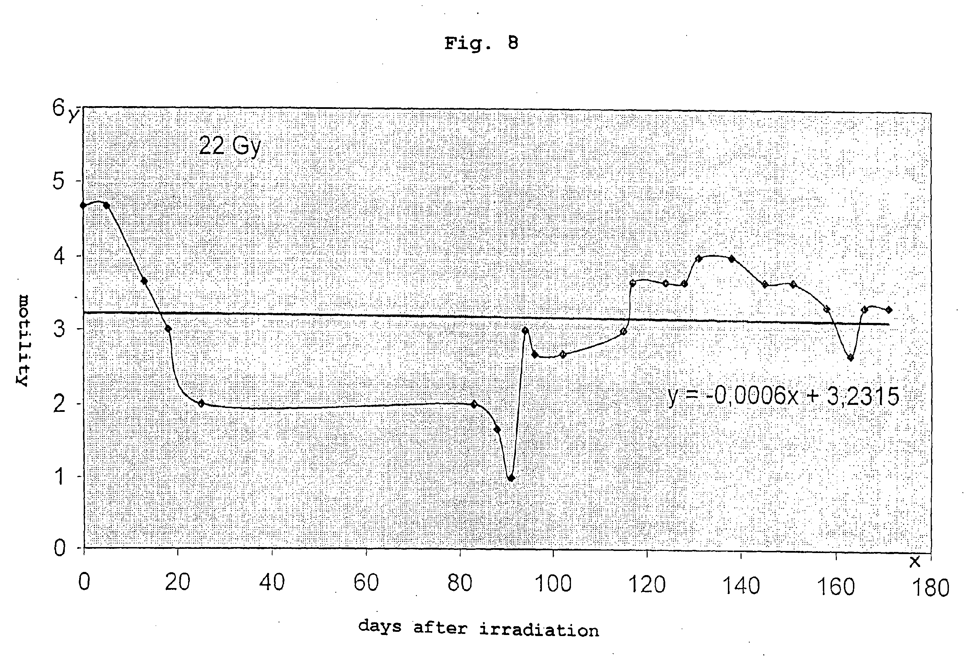

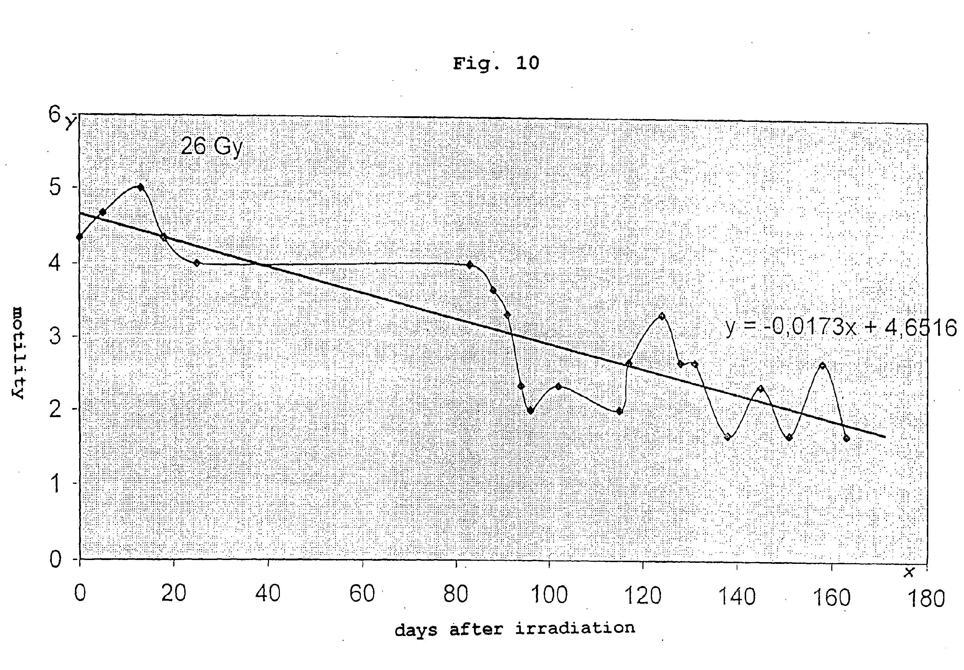

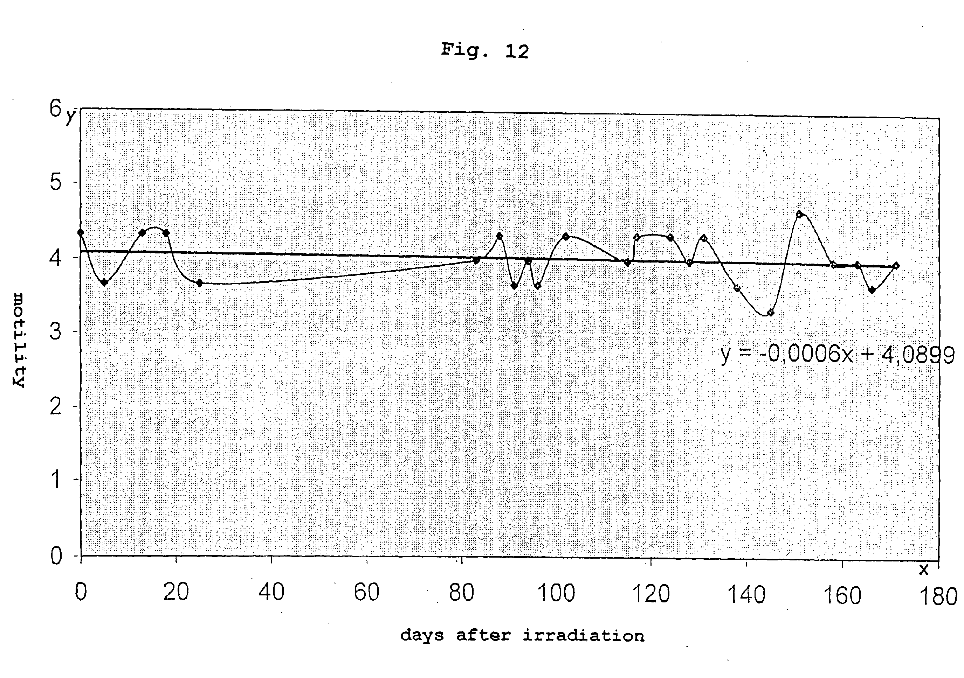

Statistically, there was no difference between the control group (Fig.12) and the groups of cocks irradiated with 18 Gy (Fig.6) and 22 Gy (Fig.8). Groups treated with a single dose of 26 Gy (Fig.10) and 5 x 8 Gy (Fig.4) doses differed significantly in their linear gradients compared with the control group and those administered with 18 Gy and 22 Gy.

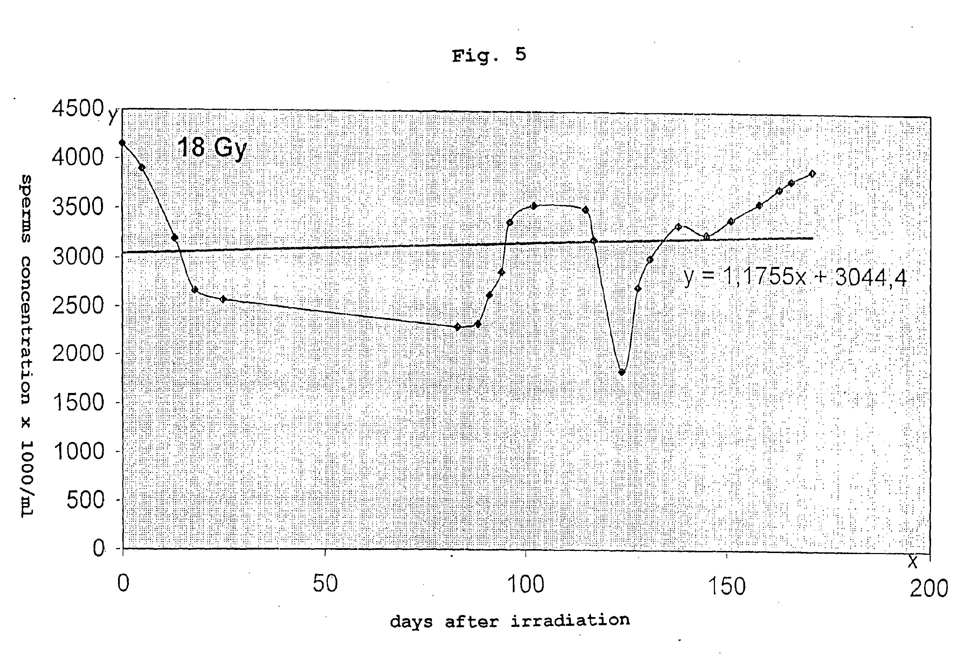

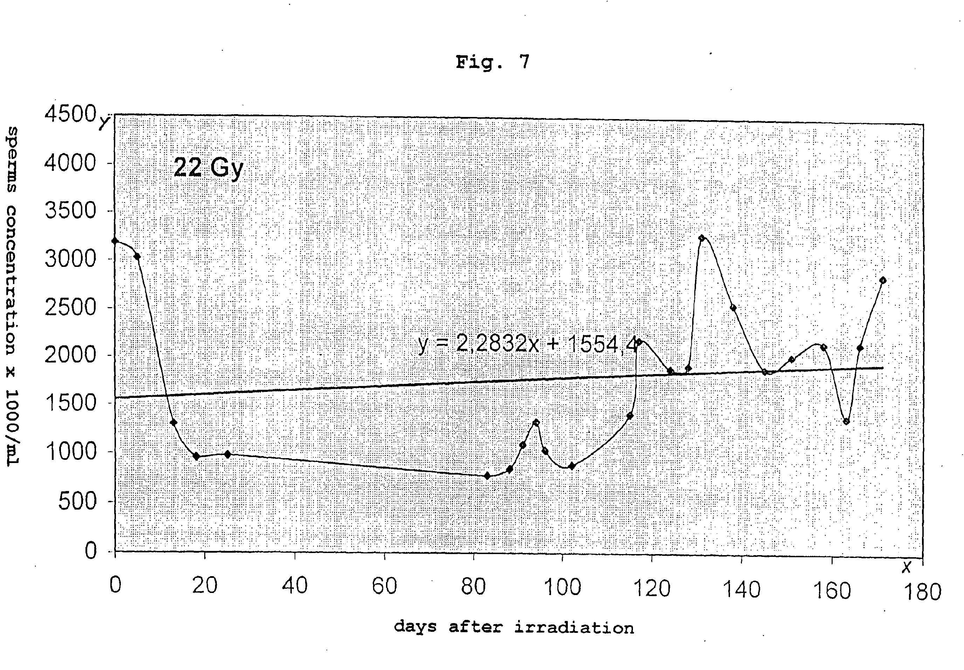

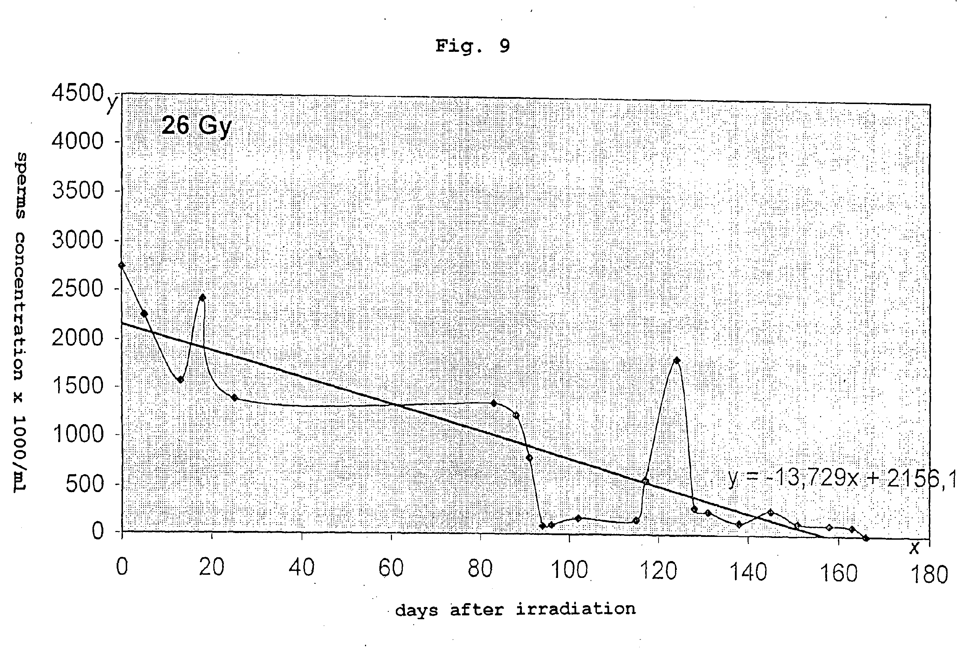

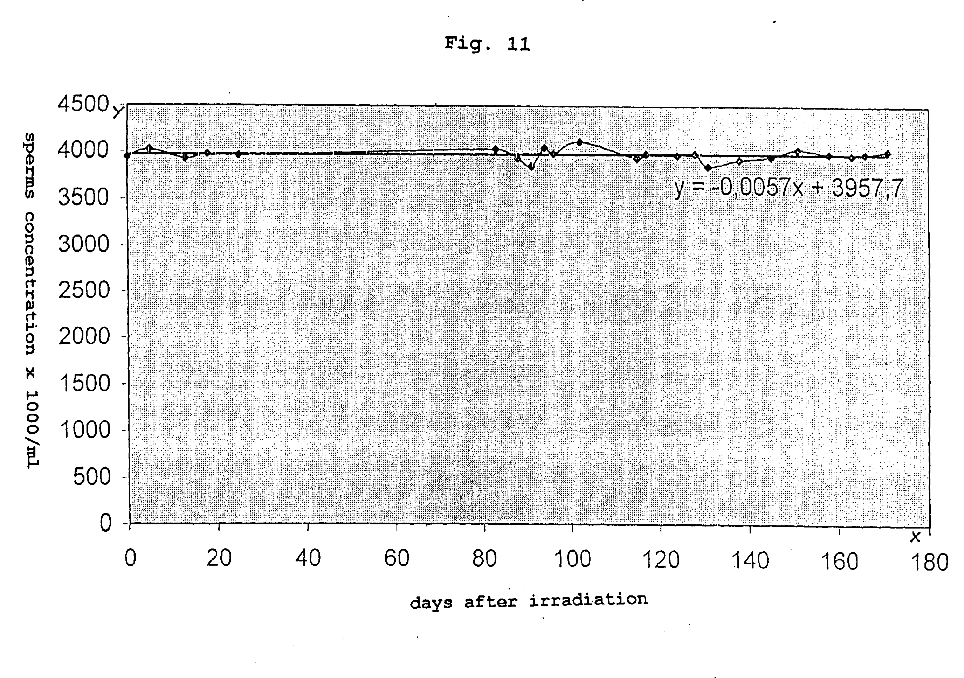

From a statistical point of view, the linear gradients of the spermatozoa concentration did not differ between the control group (Fig.11) and groups irradiated with single 18 (Fig.5) and 22Gy (Fig.7) doses. However, a significant difference was shown with the single 26Gy group (Fig.9) and the group repeatedly irradiated by 5 doses of 8Gy (Fig.3) compared to 18Gy, 22Gy and control groups.

The group treated with one dose of 26Gy never attained zero spermatozoa concentrations.

### Detailed Description of the Invention

### Example 1

### Complete sterilization of an acceptor cock:

A mature cock of the Leghorn white inbred strain (white colour of feathers is caused by the dominant allele II in locus I) producing ejaculate was fixed in a box so that it was possible to carry out an irradiation with gamma rays directed on the side of his body exactly to an area of the testicle's size. The instrument, model Theratron T1000,with isotope ⁶⁰Co was suitable for this purpose. Using this instrument testicles of this cock were irradiated 5-times, each distributed at a week (i.e.7 days) interval, by at least the absorbed dose 8 Gy. Without producing any adverse effects on the cock health and breeding condition, about 90 days after the last irradiation the cock lost the ability to produce sperms permanently and after this period his testicles were ready to be colonized by the germline spermatogonial cells produced by the donor cock, see the graphs in Fig. 3 and Fig. 4 and the photograph in Fig. 2.

### Preparation and application of germline spermatogonial cells of the donor cock:

A mature cock of the Minorca black inbred strain (black color of feathers is caused by the recessive allele ii) producing ejaculate was subjected to a tissue sampling by biopsy from his testicles which sample contained his germline spermatogonial cells. The cells were immersed into the M 199 Sigma medium (containing expressed by weight: 10 % of fetal bovine serum, 2 % of chicken serum, 1 % of pyruvate sodium, 1 % of gentamycin), whereby, the dilution ratio with this medium was 1:1. Cell incubation was carried out in standard conditions, i.e. carbon dioxide content 5 % by weight, temperature 40 °C. Spermatogonial cells could be transfected with any foreign DNA during this incubation. Thereafter, the total volume 200 microlitres of the germline spermatogonial cells suspension was applied by means of a syringe into each irradiated testicle of the acceptor cock at the time when the irradiated cock ceased to produce any ejaculate, i.e. 85 to 100 days after the last irradiation dose.

### Functional production of ejaculate after a transfer of the germline spermatogonial cells of the donor cock:

The irradiated acceptor cock of the Leghorn inbred strain with non-functional testicles started to produce ejaculate after application of foreign germline spermatogonial cells of the donor cock of the Minorca black inbred strain.

### Biological testing of the transferred germline spermatogonial cells of the donor cock:

Having carried out insemination of a hen of the Leghorn barred inbred strain (the barred color is caused by recessive allele ii by piebald gene bound to sex B/-) by ejaculate taken from the irradiated cook of the Leghorn white inbred strain, which cock is acceptor of foreign germline spermatogonial cells of the donor cock of the Minorca black inbred strain, the inseminated eggs were put into an incubator and after 21 days of incubating only black chicken hatched.

The black chicken are a sound proof that the implantation and colonization of the germline spermatogonial cells of the donor cock was successful because if only black or barred chicken hatch, in fact it indicates that the dominant allele II is not present and that only the recessive allele ii is present, i.e. that only functional sperms of the cock with recessive feather color ii are present, i.e. in this case of those with the black feather color, see Fig. 2.

### Example 2

### Incomplete sterilization of the acceptor cock:

A mature cock of the Leghorn white inbred strain (white feather colour caused by dominant allele II in locus I) producing ejaculate was fixed for irradiation in the same way as in Example 1. Testicles of this cock were irradiated by means of the irradiation instrument by a one-time absorbed dose of 18 Gy. Without deteriorating the health state and the breeding condition of the cock, the cock did not loose the ability to produce sperms permanently (in comparison to check, see the graphs in Figures 11 and 12), on the contrary, after 150 days the concentration (see the graph in Fig. 5) and the motility (see the graph in Fig. 6) returned to normal values before the irradiation and the cock testicles were not prepared to be colonized by germline spermatogonial cells of the donor cock.

### Example 3

### Incomplete sterilization of an acceptor cock:

A mature cock of the Leghorn white inbred strain (white colour of feathers is caused by the dominant' allele II in locus I) producing ejaculate was fixed in a box so as in Example 1 during irradiation. Testicles of this cock were irradiated with a one-time absorbed dose 22 Gy provided by the same irradiation instrument.

Without producing any adverse effects on the cock's health and breed condition, during 200 days of monitoring, the cock did not lose the ability to produce sperms permanently. Conversely, after 160 days, the concentration (see graph in Fig. 7) and motility (graph in Fig. 8) were a little bit lower, but they returned to the original level existing before the irradiation and the cock's testicles were not prepared to be colonized by germline spermatogonial cells of the donor cock.

### Example 4

### Incomplete sterilization of the acceptor cock:

A mature cock of the Leghorn white inbred strain (white color of feathers is caused by the dominant allele II in locus I) producing ejaculate was fixed in a box so as in Example 1 during irradiation. Testicles of this cock were irradiated with a one-time absorbed dose 26 Gy provided by the same irradiation instrument. This absorbed dose influenced health state of this cock negatively. The cock's skin was slightly swollen and reddish in the irradiation place, the cock was tired for several days after the irradiation. The cock lost ability to produce sperms not sooner then nearly after 160 days, (see graph in Fig. 9), but production of ejaculate continued at a very low level and the sperms have shown certain motility (see graph in Fig. 10). It was obvious that this absorbed dose was rather too high, it deteriorated health state of the cock, but considering that the ejaculate production continued, this absorbed dose was still insufficient and the cock testicles were not yet prepared for colonization by the germline spermatogonial cells of the donor cock.

### Industrial Use

By repeated irradiation of the acceptor cock's testicles with gamma rays and by implanting foreign germline spermatogonial cells of the donor cock into the so treated undamaged but sterile testicles of the acceptor cock, sperms able to fertilize that store all genetic information of the donor cock are repeatedly produced by this acceptor cock. After insemination of hens by the sperms so generated, transgenic fowl may be produced which fowl is provided to ½ with the genetic information of the donor cock.

Method according to the present invention will find industrial use in fowl breeding and biotechnological industries.

Production of transgenic fowl by this method appears easy because the spermatogonial cells of the donor cock can be transfected by the necessary genetic information and so after a successful implantation of said cells into testicles of a acceptor cock a transgenic cock is so produced, which cock after insemination of hens by his ejaculate or by natural breeding will pass to ½ the genetic information of the donor to his offspring.

## Claims

1. A method of sterilisation of an acceptor cock, wherein the testes are preserved but sterile, so that foreign spermatogonial germline cells from a donor cock can be transferred to said testes and can colonise them, **characterized in that** the testes only of the acceptor cock are externally irradiated 5 times over a week, each time with 8 Gy gamma rays.

2. A method according to claim 1, wherein the foreign spermatogonial germline cells from a donor cock are transfected with exogenous genetic information prior to the transfer to the sterilised testes.

## Patentansprüche

1. Verfahren zur Sterilisierung eines Empfänger-Hahns, in dem die Hoden geschont werden, jedoch steril sind, so dass fremde Spermatogonienkeimbahnzellen eines Spender-Hahns in diese Hoden übertragen werden und diese besiedeln können, **dadurch gekennzeichnet, dass** allein die Hoden des Empfängerhahns fünfmal in einem Zeitrum über eine Woche mit jeweils 8 Gy γ-Strahlen extern bestrahlt werden.

2. Verfahren gemäß Anspruch 1, wobei die fremden Spermatogonienkeimbahnzellen eines Spender-Hahns vor der Übertragung in die sterilisierten Hoden mit exogener genetischer Information transfiziert werden.

## Revendications

1. Procédé de stérilisation d'un coq receveur, dans lequel les testicules sont préservés mais stériles, de telle sorte que des cellules germinales spermatogoniales étrangères d'un coq donneur puissent être transférées auxdits testicules et puissent les coloniser, **caractérisé en ce que,** seuls les testicules du coq receveur sont irradiés de l'extérieur 5 fois pendant une semaine, avec 8 Gy de rayons gamma à chaque fois.

2. Procédé selon la revendication 1, dans lequel les cellules germinales spermatogoniales étrangères provenant d'un coq donneur sont transfectées avec une information génétique exogène avant le transfert aux testicules stérilisés.
